# EUROPEAN PATENT SPECIFICATION

(11) **EP 3 057 550 B1**
(45) Date of publication and mention of the grant of the patent: **10.07.2019**
(21) Application number: 14853546.1
(22) Date of filing: 15.10.2014
(51) Int. Cl.: A61F 11/04, H04R 25/02, A61N 1/05

(54) **DEPLOYABLE AND MULTI-SECTIONAL HEARING IMPLANT ELECTRODE**
EINSETZBARE UND AUS MEHREREN ABSCHNITTEN BESTEHENDE HÖRIMPLANTATELEKTRODE
ÉLECTRODE D'IMPLANT AUDITIF DÉPLOYABLE ET À MULTIPLES SECTIONS

(30) Priority: 15.10.2013 US 201361890923 P
(43) Date of publication of application: 24.08.2016
(73) Proprietor: Med-El Elektromedizinische Geraete GmbH, 6020 Innsbruck (AT)
(72) Inventor: LENARZ, Thomas, 30559 Hannover (DE); DHANASINGH, Anandhan, Choolaimedu, Channai-600094 (IN); JOLLY, Claude, 6020 Innsbruck (AT); HOCHMAIR, Ingeborg, 6094 Axams (AT); RIDL, Anita, 6175 Kematen (AT)
(74) Representative: Downing, Michael Philip
(86) International application number: PCT/US2014/060624
(87) International publication number: WO 2015/057794

(56) References cited:
- EP-A1- 0 971 660
- US-A- 5 667 514
- US-A- 6 151 526
- US-A1- 2005 267 549
- US-A1- 2006 212 094
- US-A1- 2007 005 117
- US-A1- 2010 069 999
- US-A1- 2011 077 660
- US-A1- 2011 319 907
- US-A1- 2012 130 465
- US-B1- 6 306 168

## Description

### FIELD OF THE INVENTION

The present invention relates to medical implants, and more specifically to an implantable electrode for use in cochlear implant systems in patients having a malformed cochlea.

### BACKGROUND ART

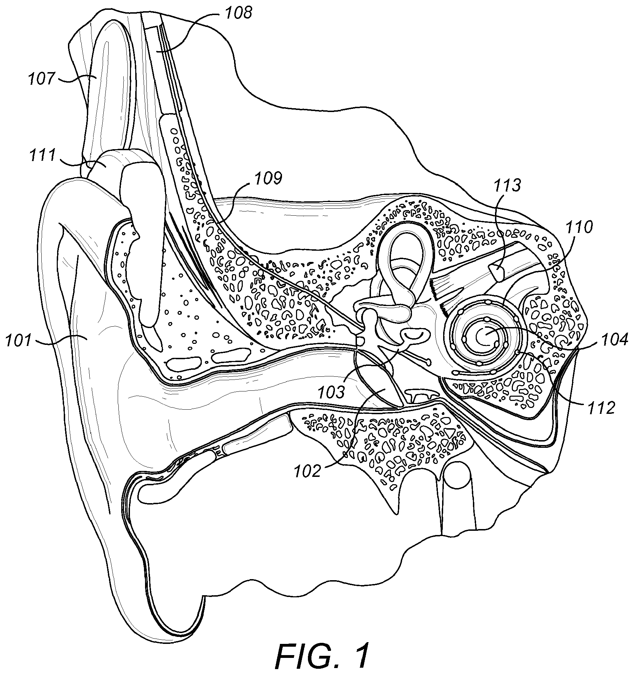

A normal ear transmits sounds as shown in Figure 1 through the outer ear **101** to the tympanic membrane (eardrum) **102,** which moves the bones of the middle ear **103** (malleus, incus, and stapes), which in turn vibrate the oval window and round window openings of the cochlea **104.** The cochlea **104** is a long narrow duct wound spirally about its axis for approximately two and a half turns. The cochlea **104** includes an upper channel known as the scala vestibuli and a lower channel known as the scala tympani, which are connected by the cochlear duct. The scala tympani forms an upright spiraling cone with a center called the modiolar where the spiral ganglion cells of the acoustic nerve **113** reside. In response to received sounds transmitted by the middle ear **103,** the fluid-filled cochlea **104** functions as a transducer to generate electric pulses that are transmitted to the cochlear nerve **113,** and ultimately to the brain.

Hearing is impaired when there are problems in the ability to transduce external sounds into meaningful action potentials along the neural substrate of the cochlea. In such cases a cochlear implant is an auditory prosthesis which uses an implanted stimulation electrode to bypass the acoustic transducing mechanism of the ear and instead stimulate auditory nerve tissue directly with small currents delivered by multiple electrode contacts distributed along the electrode.

Various devices have been proposed to address the problem of impaired hearing. US patent application US2005/0267549 discloses a system for treating patients affected by hearing loss and balance disorders resulting from vestibular hypofunction and/or malfunction. US patent US6151526 discloses an implantable stimulation device to electrically stimulate the auditory nerve comprising an electrode.

Figure 1 shows some components of a typical cochlear implant system which includes an external microphone that provides an audio signal input to an external signal processing stage **111** where various signal processing schemes can be implemented. The processed signal is then converted into a digital data format, such as a sequence of data frames, for transmission into the implant stimulator **108.** Besides extracting the audio information, the implant stimulator **108** also performs additional signal processing such as error correction, pulse formation, etc., and produces a stimulation pattern (based on the extracted audio information) that is sent through connected wires **109** to an implanted electrode carrier **110.** Typically, this electrode carrier **110** includes multiple electrodes on its surface that provide selective stimulation of the cochlea **104.**

Cochlear implant systems need to deliver electrical power from outside the body through the skin to satisfy the power requirements of the implanted portion of the system. As shown in Fig. 1, an external transmitter coil **107** (coupled to the external signal processor **111**) is placed on the skin adjacent to a subcutaneous receiver coil connected to the implant stimulator **108.** Often, a magnet in the external coil structure interacts with a corresponding magnet in the subcutaneous secondary coil structure. This arrangement inductively couples a radio frequency (rf) electrical signal to the implant stimulator **108.** The implant stimulator **108** is able to extract from the rf signal both the audio information for the implanted portion of the system and a power component to power the implanted system.

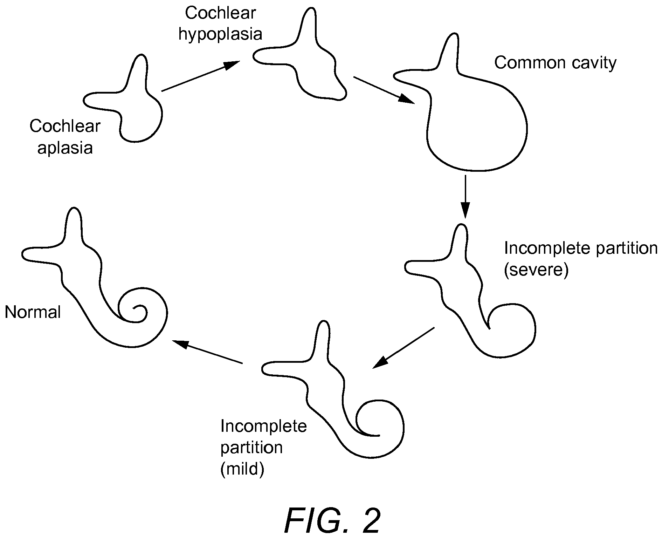

In some persons, the cochlear shape fails to develop properly and various malformation conditions can occur such as those shown in Figure 2: cochlear aplasia, cochlear hypoplasia, common cavity (CC) malformation, and incomplete partitioning. Specifically in a common cavity malformation the cochlea and the vestibule are represented by a single chamber. This structure may have cochlear and vestibular neural structures, but it completely lacks inter-scala separation (no basilar membrane), no modiolus trunk, and it appears as a single cavity. The neural structures are believed to be present at the bony capsule defining the outer cavity wall. The specific size of the cavity can vary significantly and can be measured using medical imaging.

Placing an electrode inside this common cavity is not straightforward and needs utmost care to ensure that the stimulation contacts are either touching or very close to the outer wall of the cavity. The current technique involves making two cochleostomy openings in the outer surface of the cochlea for the electrode placement, which is undesirably traumatic.

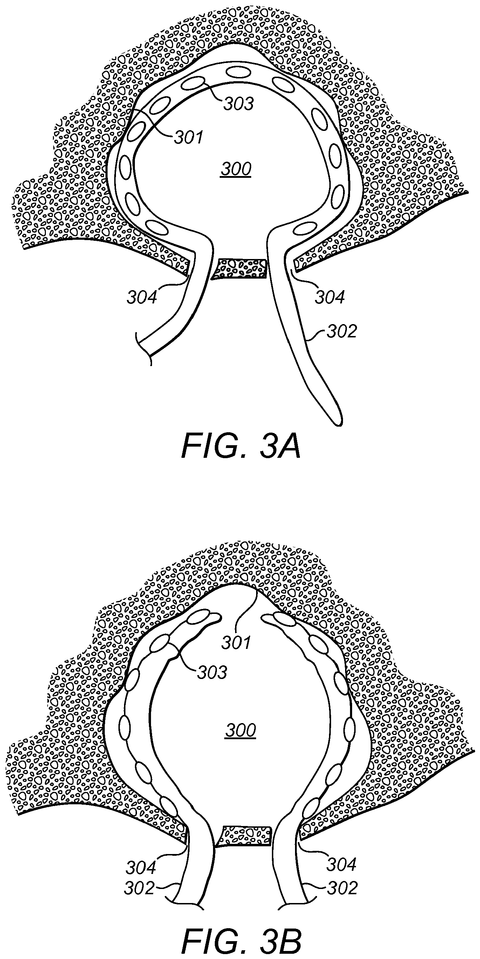

Fig. 3A shows one approach wherein the electrode array **302** has an extended distal end. Two cochleostomies **304** are made in the outer surface of the cochlea **300,** the electrode array **302** is inserted through one of the cochleostomies **304,** and the distal tip of the electrode array **302** is retrieved and pulled through the other cochleostomy **304.** The surgeon has to manipulate the electrode array **302** to attempt to place the stimulation contacts **303** against the outer wall **301** of the cavity, after which the final position of the electrode array **302** is fixed and the distal extension may be removed.

Fig. 3B shows another approach for electrode implantation in a common cavity, again requiring two cochleostomies **304** in the outer surface of the cochlea **300.** Two separate electrode arrays **302** are used, one through each cochleostomy **304,** and again considerable surgical skill is needed to manipulate the electrode arrays **302** to place their stimulation contacts **303** adjacent to the outer wall **301** of the cavity. Both techniques are highly traumatic in requiring two cochleostomies and both require considerable surgical skill to be effective.

### SUMMARY OF THE INVENTION

Embodiments of the present invention are directed to an implantable electrode for a cochlear implant patient with a malformed common cavity cochlea having a single internal cavity defined by an outer cavity wall. An extra-cochlear electrode lead contains signal wires for conducting electrical stimulation signals. An intra-cochlear electrode array is suitable for insertion into the single internal cavity of the cochlea through a single cochleostomy opening. Outer array branches arranged in an umbrella shape are closable about an array trunk having a center axis and the electrode array is configured to be closed into a closed umbrella shape for insertion through the single cochleostomy opening into the internal chamber of the cochlea. The electrode array opens into an umbrella shape within the internal cavity after insertion into the cochlea so that each outer array branch moves away from the center axis to lie with their outer surfaces against the outer cavity wall to deliver the electrical stimulation signals through a plurality of stimulation contacts on each outer array branch to adjacent neural tissue for auditory perception by the patient.

An outer insertion tube is configured to contain the folded electrode array for insertion into the cochlea, and is retractable back through the cochleostomy opening after insertion of the electrode array into the cochlea to allow the array branches to open within the internal cavity of the cochlea. In specific embodiments the insertion tube may contain a longitudinal slit along its outer surface for removal of the insertion tube from the electrode lead after retraction. The insertion tube also may include one or depth indicator marks along its outer surface.

The electrode array may be configured to be closed into a closed umbrella shape with the array branches lying along the center axis for insertion into the cochlea and to be opened into an open umbrella shape after insertion into the cochlea. Or the electrode array may be configured to be closed into a closed inverted umbrella shape with the array branches lying along the center axis for insertion into the cochlea and to be opened into an open inverted umbrella shape after insertion into the cochlea. The outer surface of each array branch may include multiple stimulation contacts, which may be on inner and/or outer surfaces.

The electrode array may be made of radio-opaque material. And the array branches have different radio-opaque patterns and/or a distal tip of the electrode array may have a radio indicator mark.

The electrode array may include a connector tip configured to hold together distal ends of the array branches, so that the array branches to move away from the center axis after the connector tip touches the outer cavity wall opposite the single cochleostomy opening during insertion of the electrode array into the cochlea. The connector tip may be permanently connected to the distal ends of the array branches, or it may be removable from the distal ends of the array branches after insertion of the electrode array into the cochlea.

Embodiments also include a complete cochlear implant system having an electrode array according to any of the above.

### BRIEF DESCRIPTION OF THE DRAWINGS

Figure 1 shows elements of a human ear having a typical cochlear implant system.
Figure 2 illustrates various cochlear malformation shapes.
Figures 3 A-B show conventional electrode insertion into a common cavity cochlea using two cochleostomies.
Figures 4 A-B show a common cavity electrode having an umbrella shape according to an embodiment of the present invention.
Figures 5 A-E show various aspects of a common cavity electrode having an inverted umbrella shape according to an embodiment of the present invention.
Figures 6 A-B show an outer insertion tube around the electrode array according to an embodiment of the present invention.
Figures 7 A-B show electrode arrays according to embodiments of the present invention after insertion into the common cavity.
Figures 8 A-B show another embodiment of a common cavity electrode.
Figures 9 A-B show another embodiment of a common cavity electrode.
Figures 10 A-B show another embodiment of a common cavity electrode.
Figures 11 A-D show an embodiment of a cochlear implant electrode with foldable array branches for a normal cochlear anatomy or a cochlea with incomplete partition (e.g., Mondini dysplasia) with contacts to both sides.
Figures 12 A-B show another embodiment of a common cavity electrode.

### DETAILED DESCRIPTION OF SPECIFIC EMBODIMENTS

Various embodiments of the present invention are directed to an implantable electrode for a common cavity cochlea having a foldable electrode array configured for insertion into the cochlea through a single cochleostomy. After entering the common cavity, the electrode array is unfolded to place array branches with the stimulating contacts adjacent to the outer cavity wall. Because the electrode is configured for insertion through a single cochleostomy opening rather than requiring two cochleostomies as in existing conventional arrangements, the amount of trauma to the cochlea is reduced and an easier surgical insertion process can be used. Further, the probability to make contact with neural elements is increased, which location and distribution inside malformed cochleae cannot be predicted by preoperative examinations.

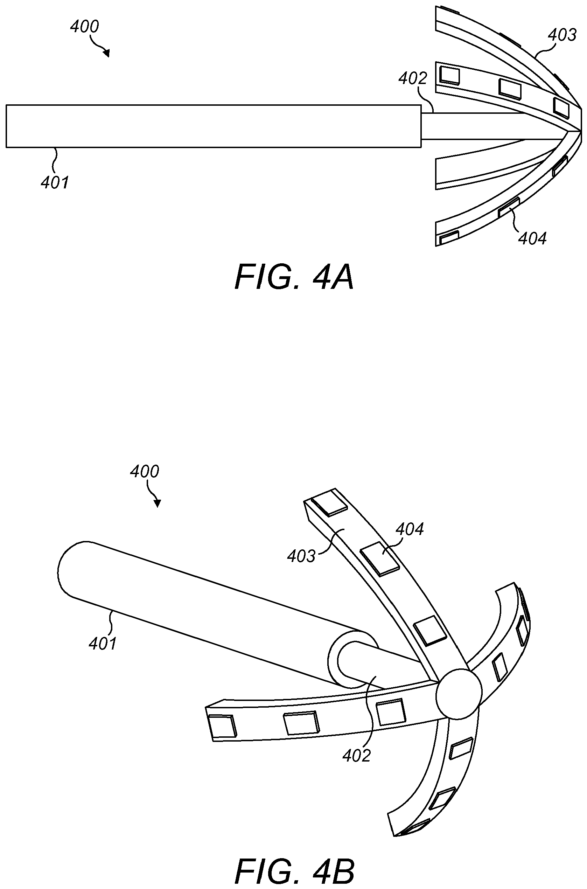

Figure 4 A-B show a common cavity electrode **400** having an umbrella shape according to an embodiment of the present invention. The common cavity electrode **400** includes an extra-cochlear electrode lead **401** that contains the signal wires that conduct the electrical stimulation signals. An intra-cochlear electrode array includes a central array trunk **402** that is elongated along a center axis and that contains the signal wires. Multiple outer array branches **403** of flexible bio-compatible polymer material are foldable about the center axis. The array branches **403** as shown in Fig 4 A-B are foldable into an umbrella shape to lie against the array trunk **402** for insertion into the cochlea. The array branches **403** have multiple stimulation contacts **404,** which may specifically be on the inner and/or outer surfaces of the array branches **403.** The electrode array branches **403** and/or the array trunk **402** may be made of radio-opaque material, and there may be a radio indicator mark on the distal tip of the array trunk **402** for medical imaging systems. Alternatively, only portions of the array branches **403** may be made of radio-opaque material such that they have different radio-opaque patterns and consequently they differ from each other on the image, e.g. an X-ray image. The knowledge about the exact placement of the individual array branches **403** may be helpful for later fitting of the device to the patient's needs and to avoid misplacement. In further embodiments there may also be stimulation contacts on the array trunk **402.**

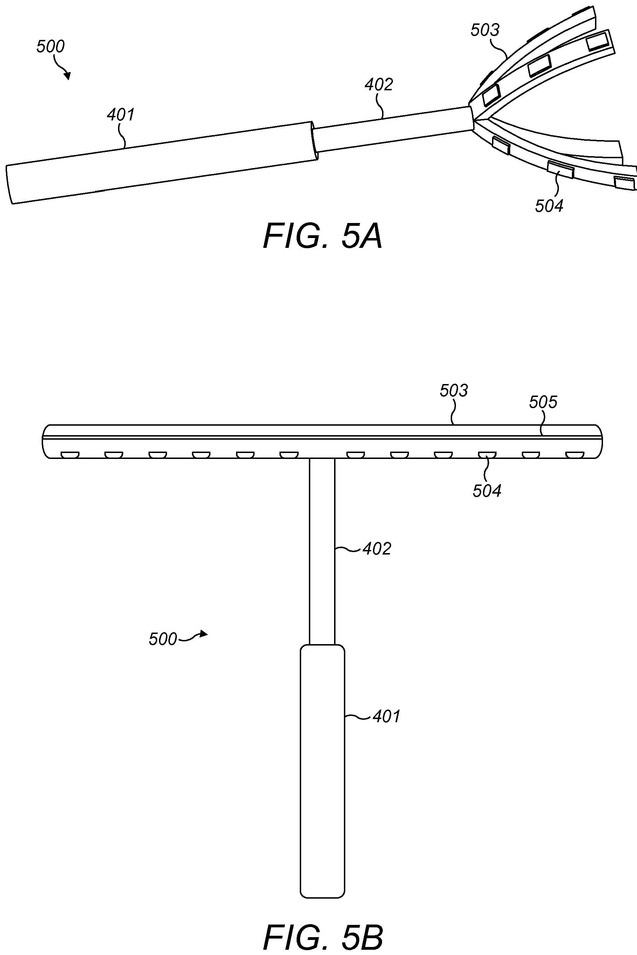

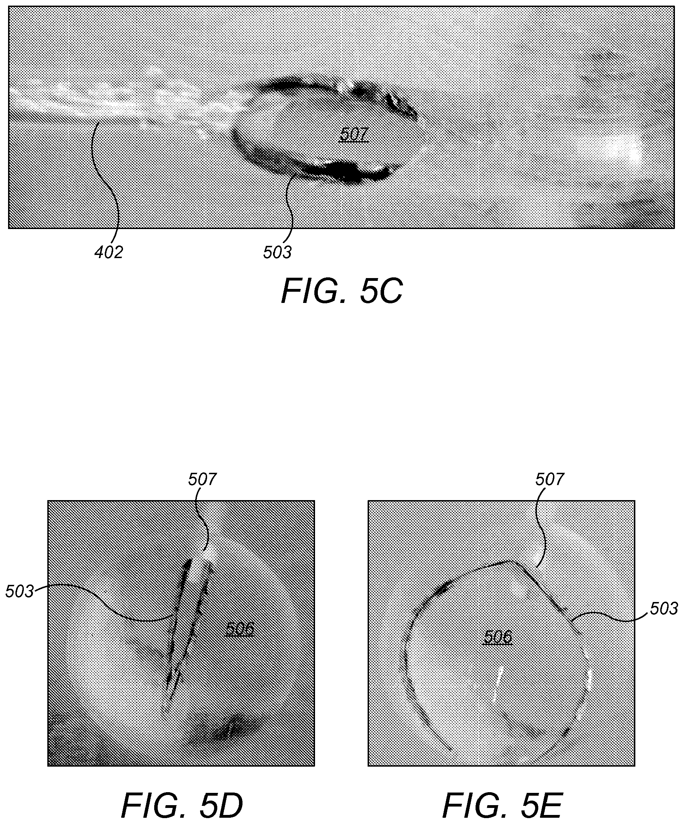

Figure 5 A shows an alternative embodiment of a common cavity electrode **500** that is foldable into an inverted umbrella shape, with four array branches **503** lying along the center axis forward of the array trunk **402** and the stimulation contacts **504** on the outer surfaces of the array branches **503.** Figure 5 B shows a similar embodiment of a common cavity electrode **500** with two opposing array branches **503** lying along the center axis forward of the array trunk **402** and the stimulation contacts **504** on the outer surfaces of the array branches **503.** A springy nitinol support wire **505** is embedded within the opposing array branches **503** as shown. In a specific embodiment, the array branches **503** might typically have a diameter of 0.3 mm and the internal support wire might have a diameter of 0.04 mm. To insert the common cavity electrode **500** into the cochlea, the array branches **503** are folded inward towards the center axis and the distal ends pushed through the single cochleostomy opening **507,** as shown in Figure 5 C. As shown in Figure 5 D, once the array branches **503** are fully within the cochlear cavity **506,** the support rod springs them outward towards the outer wall of the cochlear cavity **506** where the stimulation contacts will be closely adjacent to the neural tissue in the outer wall.

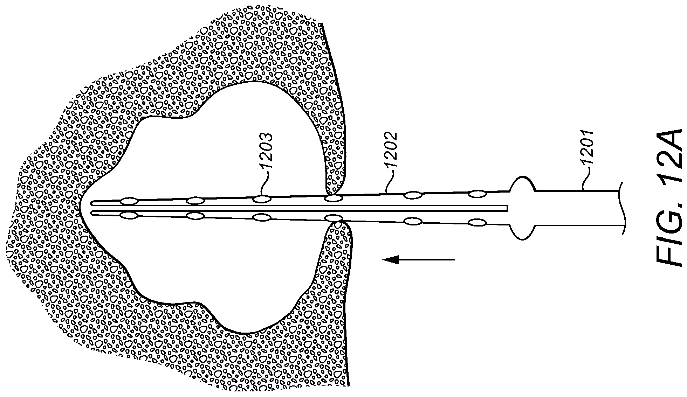

Figure 12 A-B shows a related embodiment where there are two array branches **1202** which together are about the same size and shape as a conventional cochlear implant electrode array. The array branches **1202** are separated by a longitudinal slit between them along the center axis while the extra-cochlear array trunk **1201** is a single larger branch. The array branches **1202** are advanced through the single cochleostomy opening into the cochlea, and once their distal ends reach the opposite cavity wall, continued pushing naturally bends the array branches **1202** out towards the outer walls of the cochlear cavity. When the array branches **1202** are fully inserted, the stimulation contacts **1203** will lie adjacent to the neural tissues in the outer walls of the cochlear cavity.

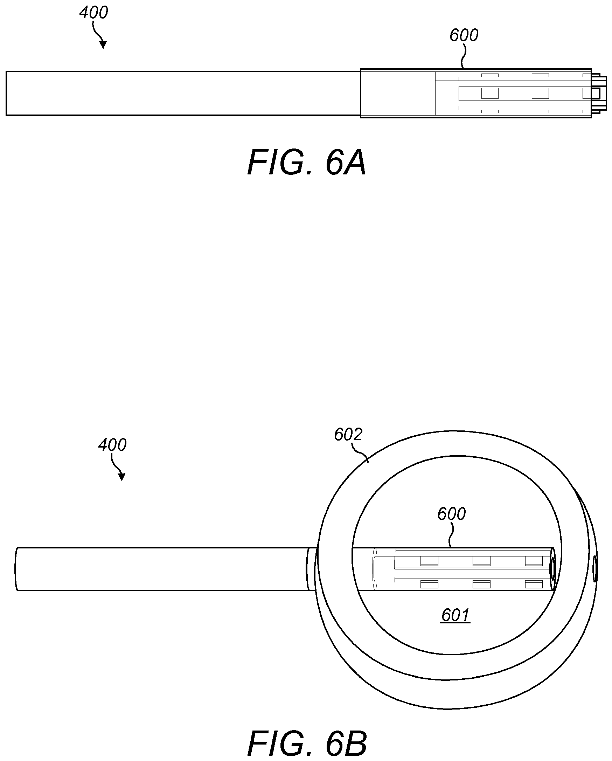

Fig. 6 A shows a common cavity electrode **400** with the array branches **403** folded against the array trunk **402** and fitted within an outer insertion tube **600** made of bio-compatible polymer material for insertion through a single cochleostomy opening in the outer surface of the cavity wall **602** and into the internal cavity of the cochlea **601** as shown in Fig. 6B. In some embodiments the insertion tube **600** may contain a longitudinal slit along its outer surface to remove it from the electrode lead **401** after retraction back outside the cochlea **601.** The insertion tube **600** also may include one or depth indicator marks along its outer surface to help the surgeon determine when the electrode array has been fully inserted. Alternatively or in addition, array trunk **402** may include such indicator mark(s).

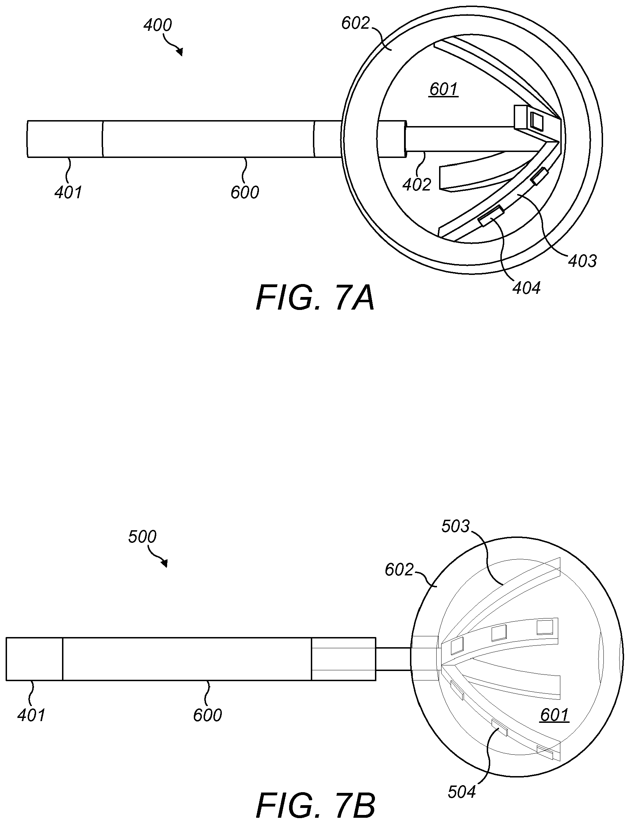

After insertion and retraction of the insertion tube **600,** the umbrella-shaped array branches **403** unfold back away from the array trunk **402,** as shown in Fig. 7 A to lie within the internal cavity **601** with their outer surfaces against the outer cavity wall **602** to deliver the electrical stimulation signals through the array branch stimulation contacts **404** to adjacent neural tissue for auditory perception by the patient. Fig. 7B shows the same arrangement for an inverted umbrella shape where the array branches **503** unfold within the internal cavity **601** away from the center axis of the electrode array to place the stimulation contacts **504** adjacent to the outer cavity wall **602.**

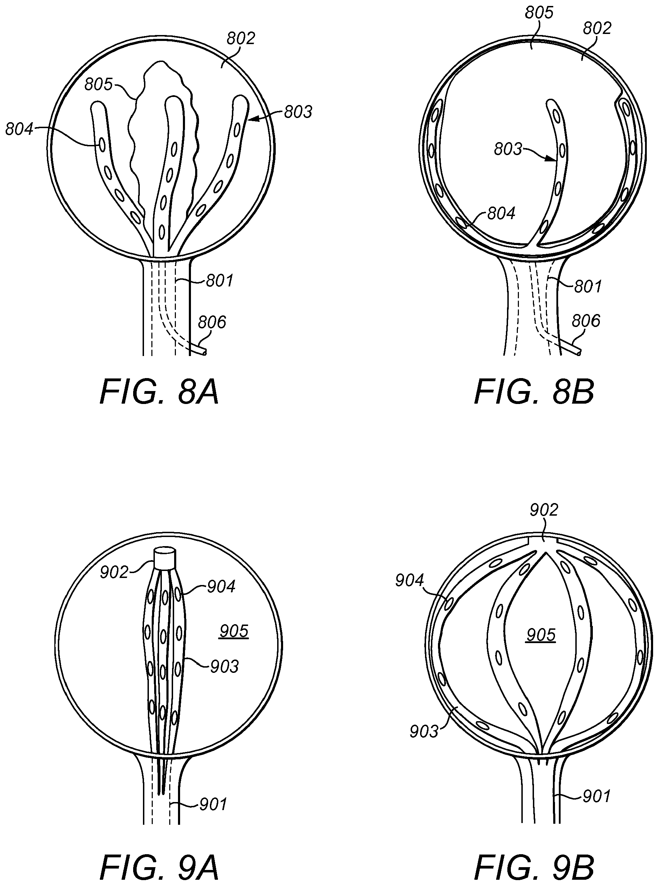

Fig. 8 A-B shows another embodiment of a common cavity electrode. After the array branches **803** have been inserted through the cochleostomy opening into the cochlea **802,** an inflation line **806** inflates an expansion balloon **805** that gently pushes the array branches **803** out in a controlled manner until the stimulation contacts **804** lie against the neural tissues at the outer cavity wall.

The inflation balloon **805** may be made of resilient silicone material and/or some or all of the inflation balloon may be made of biodegradable material. Air, gas or biocompatible liquid may be used to fill the inflation balloon **805.** In some embodiments, therapeutic substances may be added to the inflation fluid which may be released after surgery (e.g., by decomposition of the biodegradable elements of the balloon) to help the cochlear tissues heal. The amount of inflation fluid needed to inflate the inflation balloon **805** may be determined prior to the implantation surgery, for example, by medical imaging.

The inflation line **806** may be an internal lumen within the electrode lead **801** and/or the expansion balloon **805** may provide a lasting connection between the array branches **803** after the balloon has been inflated. In that case, a sealing mechanism such as a self-sealing membrane should be provided to tightly close the inflation line **806** after the inflation balloon is filled in order to prevent the ingress of bacteria into the cochlea **802.** Or the inflation line **806** and expansion balloon **805** may be separate elements from the rest of the electrode. In the latter case, it may be possible to deflate the expansion balloon **805** after the array branches **803** have been deployed, and withdraw the expansion balloon **805** and inflation line **806** from the cochlea **802.**

Figure 9 A-B shows another embodiment of a common cavity electrode with a silicone connector tip **902** at the distal end of the array branches **903.** During implantation, the electrode is pushed through the cochleostomy opening into the interior common cavity of the cochlea **905** until the connector tip **902** touches the outer wall of the cavity opposite the cochleostomy opening. The surgeon continues to push the electrode lead **901** towards the cochleostomy opening causing the array branches **903** to bend outward towards the outer cavity wall of the cochlea **905** until the stimulation contacts **904** lie against the neural tissues at the outer cavity wall.

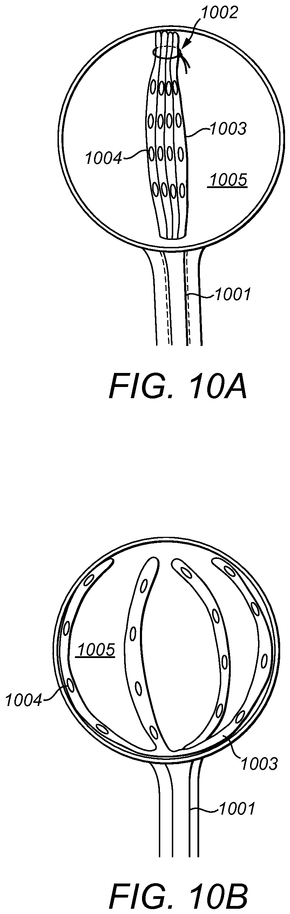

Figure 10 A-B shows a related embodiment with a temporary tip connection **1002** that allows the array branches **1003** to bend out towards the outer cavity wall of the cochlea **1005** until the stimulation contacts **1004** lie against the neural tissues there. The tip connection **1002** may be a biodegradable wire or filament (e.g., dissolving suture material) which dissolves after a period of time following surgery. Or the tip connection **1002** may be loose enough around the distal tips of the array branches **1003** that it can slip off or be untied after insertion through the cochleostomy opening.

The common electrode arrangements described above cover the distribution of neural structures in a common cavity much better than conventional existing electrodes (e.g., as shown in Figs. 3 A-B). The common cavity is a three-dimensional volume whereas the existing conventional electrodes of the type shown in Figs. 3A-B are basically two-dimensional structures. By contrast, the common cavity electrodes described above can better cover all three dimensions of the common cavity.

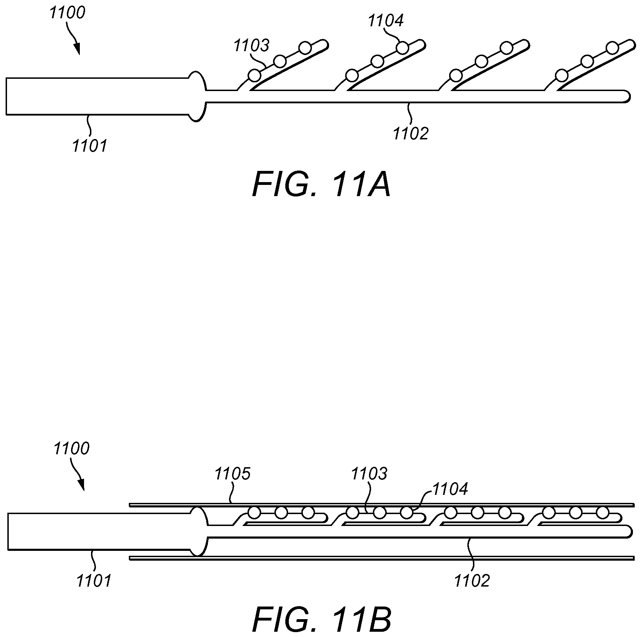

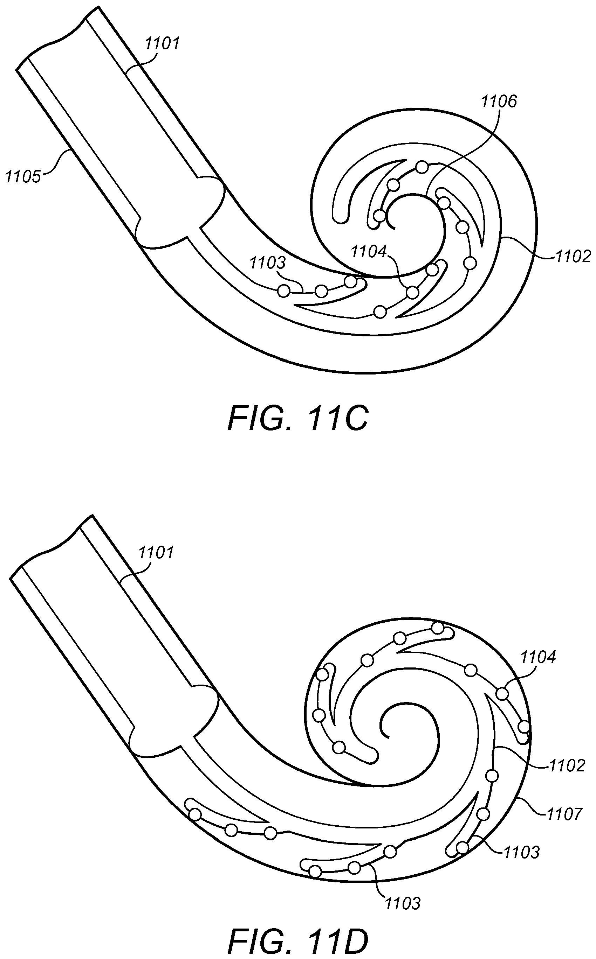

The approach of foldable array branches as described above may also be useful in a cochlear implant electrode for insertion into a conventionally spiral-shaped cochlea or cochlea with incomplete partition, such as in Mondini's dysplasia. In Mondini's dysplasia, the cochlea and the vestibular organ is separated, but in the cochlea, only the basal turn is normally formed leaving the middle and the apical turn to appear as a single cyst, resulting in an incomplete partition. For example, Figures 11 A-D show an embodiment of a cochlear implant electrode **1100** for a normal spiral-shaped cochlear anatomy (Fig. 11C) or a cochlea anatomy with incomplete partition (Fig. 11D) with foldable array branches **1103** that lie against the array trunk **1102** during implantation surgery. As shown in Fig. 11B, this may be by means of an outer insertion tube **1105** that fits over the folded array branches **1103** and array trunk **1102** for insertion into the cochlea. Once the array has been fully inserted, the insertion tube **1105** is slid back out of the cochlea over the extra-cochlear electrode lead **1101,** allowing the array branches **1103** to fold outward to place the stimulation contacts against the inner modiolar wall **1106** to gently engage the neural tissues there, as shown in Fig. 11C, or against the outer modiolar wall **1107** to engage the neural tissues there, as shown in Fig. 11D. Alternatively, the array branches **1103** may be included on both sides of the array trunk **1102,** so that the stimulation contacts **1004** may engage both the outer and inner walls **1106, 1107.** The array branches **1103,** whether positioned with the array branches **1103** facing the outer modiolar wall, the inner modiolar wall, or both, may be fixed to the array trunk **1102** at either their proximal end (such as shown in Fig. 11C) or distal ends (such as shown in Fig. 11D). If the electrode **1100** is inserted and then slightly retracted, the array branches **1103** may separate more easily when the array branches are fixed at their distal ends, as shown in Fig. 11D.

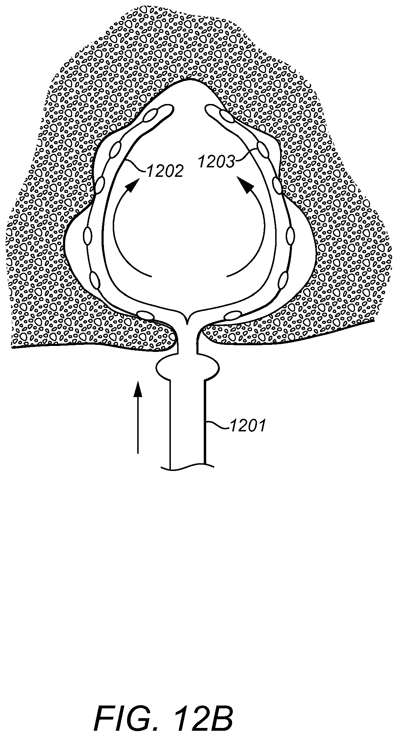

Figures 12 A-B show another embodiment of a common cavity electrode that includes a double branch electrode having electrode contacts **1203** on the individual branches that are preferably arranged at the lateral side of the electrode arrays **1202.** This arrangement supports the idea of having a single cochleostomy. In this embodiment, the electrode arrays **1202** are inserted through the cochleostomy into the common cavity until a point opposite the cochleostomy, as shown in Fig. 12A, the two branches of the electrode arrays **1202** will then move apart from each other and contact the wall of the cochlea, as shown in Fig. 12B. During the insertion process of the common cavity electrode, an insertion tube, such as described above, may be used and positioned around the double branch arrangement during the first phase of insertion. When the tips of the electrode arrays **1202** touch the cochlea opposite the cochleostomy, the insertion tube may be retracted in a coordinated manner together with further insertion of the electrode arrangement.

The present invention is set out in the appended claims.

## Claims

1. An implantable electrode for a cochlear implant patient with cochlea having a single internal cavity defined by an outer cavity wall, the electrode comprising:
an extra-cochlear electrode lead (401) containing a plurality of signal wires for conducting electrical stimulation signals; and
an intra-cochlear electrode array suitable for insertion into the single internal cavity of the cochlea, the electrode array comprising a plurality of outer array branches (403) arranged in an umbrella shape that is closable about an array trunk (402) having a center axis, each outer array branch having an outer surface with a plurality of stimulation contacts (404) for delivering the electrical stimulation signals to adjacent neural tissue;
wherein the electrode array and the array branches are configured to be closed into a closed-umbrella shape for insertion into the cochlea through a single cochleostomy opening, and
to be opened into an open umbrella shape within the internal cavity after insertion into the cochlea with the array branches away from the center axis lying with their outer surfaces against the outer cavity wall of the single internal cavity for delivery of the electrical stimulation signals through the stimulation contacts to adjacent neural tissue for auditory perception by the patient.

2. The implantable electrode according to claim 1, further comprising:
an outer insertion tube (600) configured to contain the closed electrode array for insertion into the cochlea, and retractable back through the cochleostomy opening after insertion of the electrode array into the cochlea to allow the array branches to open within the internal cavity of the cochlea.

3. The implantable electrode according to claim 2, wherein the insertion tube contains a longitudinal slit along its outer surface for removal of the insertion tube from the electrode lead after retraction.

4. The implantable electrode according to claim 2, wherein the insertion tube includes one or depth indicator marks along its outer surface.

5. The implantable electrode according to claim 1, wherein each array branch has stimulation contacts on both inner and outer surfaces.

6. The implantable electrode according to claim 1, wherein the electrode array is made of radio-opaque material.

7. The implantable electrode according to claim 6, wherein the array branches have different radio-opaque patterns.

8. The implantable electrode according to claim 6, wherein a distal tip of the electrode array has a radio indicator mark.

9. The implantable electrode according to claim 1, wherein the electrode array includes a connector tip configured to hold together distal ends of the array branches, and wherein the electrode array is configured for the array branches to move away from the center axis after the connector tip touches the outer cavity wall opposite the single cochleostomy opening during insertion of the electrode array into the cochlea.

10. The implantable electrode according to claim 9, wherein the connector tip is permanently connected to the distal ends of the array branches.

11. The implantable electrode according to claim 9, wherein the connector tip is configured to be removed from the distal ends of the array branches after insertion of the electrode array into the cochlea.

12. A cochlear implant system having an implantable electrode according to any of claims 1-11.

## Patentansprüche

1. Implantierbare Elektrode für einen Cochlea-Implantat-Patienten mit einer Cochlea, die einen einzigen internen Hohlraum aufweist, der durch eine äußere Hohlraumwand definiert ist, wobei die Elektrode Folgendes umfasst:
eine Extracochlea-Elektrodenleitung (401), die mehrere Signaldrähte zum Leiten elektrischer Stimulationssignale enthält; und
ein Intracochlea-Elektrodenarray, das zum Einsetzen in den einzigen internen Hohlraum der Cochlea geeignet ist, wobei das Elektrodenarray mehrere Außenarrayzweige (403) umfasst, die in einer Regenschirmform, die schließbar ist, um einen Arraystamm (402) mit einer Mittelachse angeordnet sind, wobei jeder Außenarrayzweig eine Außenoberfläche mit mehreren Stimulationskontakten (404) zum Liefern der elektrischen Stimulationssignale an angrenzendes neurales Gewebe aufweist;
wobei das Elektrodenarray und die Arrayzweige dazu konfiguriert sind, zum Einsetzen in die Cochlea durch eine einzige Cochleostomieöffnung in eine Geschlossener-Regenschirm-Form geschlossen zu werden, und
nach dem Einsetzen in die Cochlea in eine Offener-Regenschirm-Form innerhalb des internen Hohlraums geöffnet zu werden, wobei die Arrayzweige von der Mittelachse weg mit ihren Außenoberflächen an der äußeren Hohlraumwand des einzigen internen Hohlraums anliegen, um die elektrischen Stimulationssignale durch die Stimulationskontakte angrenzend an neurales Gewebe zur Hörwahrnehmung durch den Patienten zu liefern.

2. Implantierbare Elektrode nach Anspruch 1, die ferner Folgendes umfasst:
ein äußeres Einführungsrohr (600), das dazu konfiguriert ist, das geschlossene Elektrodenarray zum Einsetzen in die Cochlea zu enthalten, und durch die Cochleostomieöffnung nach dem Einsetzen des Elektrodenarrays in die Cochlea zurückziehbar ist, um zu ermöglichen, dass sich die Arrayzweige innerhalb des internen Hohlraums der Cochlea öffnen.

3. Implantierbare Elektrode nach Anspruch 2, wobei das Einführungsrohr einen Longitudinalschlitz entlang seiner Außenoberfläche zum Entfernen des Einsetzungsrohres von der Elektrodenleitung nach einem Rückzug enthält.

4. Implantierbare Elektrode nach Anspruch 2, wobei das Einführungsrohr ein oder mehrere Tiefenindikatormarkierungen entlang seiner Außenoberfläche beinhaltet.

5. Implantierbare Elektrode nach Anspruch 1, wobei jeder Arrayzweig Stimulationskontakte sowohl auf Innenals auch Außenoberflächen aufweist.

6. Implantierbare Elektrode nach Anspruch 1, wobei das Elektrodenarray aus funkundurchlässigem Material gefertigt ist.

7. Implantierbare Elektrode nach Anspruch 6, wobei die Arrayzweige unterschiedliche funkundurchlässige Strukturen aufweisen.

8. Implantierbare Elektrode nach Anspruch 6, wobei eine distale Spitze des Elektrodenarrays eine Funkindikatormarkierung aufweist.

9. Implantierbare Elektrode nach Anspruch 1, wobei das Elektrodenarray eine Verbinderspitze beinhaltet, die dazu konfiguriert ist, die distalen Enden der Arrayzweige zusammenzuhalten, und wobei das Elektrodenarray so konfiguriert ist, dass sich die Arrayzweige von der Mittelachse weg bewegen, nachdem die Verbinderspitze die äußere Hohlraumwand gegenüber der einzigen Cochleostomieöffnung während einer Einsetzung des Elektrodenarrays in die Cochlea berührt hat.

10. Implantierbare Elektrode nach Anspruch 9, wobei die Verbinderspitze permanent mit den distalen Enden der Arrayzweige verbunden ist.

11. Implantierbare Elektrode nach Anspruch 9, wobei die Verbinderspitze dazu konfiguriert ist, von den distalen Enden der Arrayzweige nach einer Einsetzung des Elektrodenarrays in die Cochlea entfernt zu werden.

12. Cochlea-Implantat-System mit einer implantierbaren Elektrode nach einem der Ansprüche 1-11.

## Revendications

1. Électrode implantable pour un patient porteur d'un implant cochléaire ayant une cochlée dont la cavité interne unique est définie par une paroi de cavité externe, l'électrode comprenant :
un fil d'électrode extracochléaire (401) contenant une pluralité de fils de signaux pour conduire des signaux de stimulation électrique ; et
un réseau d'électrode intracochléaire adapté à une introduction dans la cavité interne unique de la cochlée, le réseau d'électrode comprenant une pluralité de branches externes (403) de réseau agencées en forme de parapluie pouvant être fermé autour d'un tronc (402) de réseau possédant un axe central, chaque branche externe de réseau possédant une surface externe dotée d'une pluralité de contacts de stimulation (404) pour administrer les signaux de stimulation électrique à un tissu neuronal adjacent ;
dans laquelle le réseau d'électrode et les branches de réseau sont conçus pour être fermés en forme de parapluie fermé pour une introduction dans la cochlée à travers une ouverture de cochléostomie unique, et
pour être ouverts en une forme de parapluie ouvert dans la cavité interne après l'introduction dans la cochlée, les branches de réseau éloignées de l'axe central reposant avec leurs surfaces externes contre la paroi de cavité externe de la cavité interne unique pour l'administration de signaux de stimulation électrique au moyen des contacts de stimulation au tissu neuronal adjacent pour une perception auditive par le patient.

2. Électrode implantable selon la revendication 1, comportant en outre :
un tube d'introduction externe (600) conçu pour contenir le réseau d'électrodes fermé pour une introduction dans la cochlée, et pouvant être replié à travers l'ouverture de cochléostomie après l'introduction du réseau d'électrode dans la cochlée pour permettre que les branches de réseau s'ouvrent dans la cavité interne de la cochlée.

3. Électrode implantable selon la revendication 2, dans laquelle le tube d'introduction contient une fente longitudinale le long de sa surface externe pour un retrait du tube d'introduction depuis le fil d'électrode après le repliement.

4. Électrode implantable selon la revendication 2, dans laquelle le tube d'introduction comprend un ou plusieurs repères de profondeur le long de sa surface externe.

5. Électrode implantable selon la revendication 1, dans laquelle chaque branche de réseau possède des contacts de stimulation à la fois sur les surfaces interne et externe.

6. Électrode implantable selon la revendication 1, dans laquelle le réseau d'électrode est en matériau radio-opaque.

7. Électrode implantable selon la revendication 6, dans laquelle les branches de réseau ont différents motifs radio-opaques.

8. Électrode implantable selon la revendication 6, dans laquelle une pointe distale du réseau d'électrode possède un repère radio-indicateur.

9. Électrode implantable selon la revendication 1, dans laquelle le réseau d'électrode comprend une pointe de connecteur conçue pour tenir ensemble des extrémités distales des branches de réseau, et dans laquelle le réseau d'électrode est conçu pour que les branches de réseau s'éloignent de l'axe central après que la pointe de connecteur a touché la paroi de cavité externe à l'opposé de l'ouverture de cochléostomie unique pendant l'introduction du réseau d'électrode dans la cochlée.

10. Électrode implantable selon la revendication 9, dans laquelle la pointe de connecteur est connectée en permanence aux extrémités distales des branches de réseau.

11. Électrode implantable selon la revendication 9, dans laquelle la pointe de connecteur est conçue pour être retirée des extrémités distales des branches de réseau après l'introduction du réseau d'électrode dans la cochlée.

12. Système d'implant cochléaire possédant une électrode implantable selon l'une quelconque des revendications 1 à 11.
